Europäisches Patentamt

European Patent Office

Office européen des brevets

(19)

(11) Veröffentlichungsnummer: **0 023 013**
**B1**

(12) **EUROPÄISCHE PATENTSCHRIFT**

(45) Veröffentlichungstag der Patentschrift:
14.07.82

(21) Anmeldenummer: 80104101.3

(22) Anmeldetag: 15.07.80

(51) Int. Cl.³: **C 03 C 12/00**, C 03 C 3/04,
A 61 K 6/06, A 61 K 6/08,
C 08 L 35/00

(54) **Calciumaluminiumfluorosilikatglas-Pulver und seine Verwendung.**

(30) Priorität: 18.07.79 DE 2929121

(43) Veröffentlichungstag der Anmeldung:
28.01.81 Patentblatt 81/4

(45) Bekanntmachung des Hinweises auf die Patenterteilung:
14.07.82 Patentblatt 82/28

(84) Benannte Vertragsstaaten:
AT BE CH DE FR GB IT LI NL SE

(56) Entgegenhaltungen:
FR-A-1 115 362
GB-A-1 532 955
US-A-3 923 688
US-A-3 972 721
US-A-4 137 086

(73) Patentinhaber: Espe Fabrik pharmazeutischer Präparate
GmbH, D-8031 Seefeld / Obb (DE)

(72) Erfinder: Schmitt, Werner, Dr., Prinzenweg 10,
D-8130 Starnberg (DE)
Erfinder: Purrmann, Robert, Dr.,
Riemerschmidstrasse 18, D-8130 Starnberg (DE)
Erfinder: Jochum, Peter, Dr., Pointweg 5,
D-8031 Hechendorf (DE)
Erfinder: Gasser, Oswald, Dr., Hartstrasse 13,
D-8031 Seefeld (DE)

(74) Vertreter: Abitz, Walter, Dr.-Ing. et al, Abitz, Morf,
Gritschneder P.O. Box 86 01 09, D-8000 München 86 (DE)

## 0 023 013

### Calciumaluminiumfluorosilikatglas-Pulver und seine Verwendung

Die Erfindung betrifft Glaspulver, die in medizinisch oder zahnmedizinisch verwertbaren Zementen, beispielsweise in Knochen- oder Dentalzementen, insbesondere dem sogenannten Glasionomerzement, eingesetzt werden können.

Glasionomerzemente werden in der DE-A 2 061 513 beschrieben. Sie bestehen aus einem Calciumaluminiumfluorosilikatglas-Pulver und einer Anmischflüssigkeit, welche allgemein als wäßrige Lösung einer Polycarbonsäure bezeichnet werden kann. Die daraus resultierende Masse kann beispielsweise als permanentes Füllungsmaterial in der Zahnheilkunde verwendet werden. Seine Bedeutung liegt darin, daß es als erstes Zahnfüllmaterial in kosmetischer und mechanischer Hinsicht befriedigt und gleichzeitig physiologisch so unbedenklich ist, daß es ohne Unterfüllungen und ähnliche Vorkehrungen direkt in den Zahn eingebracht werden kann.

Ein Nachteil der Glasionomerzemente ist ihre hohe Wasserempfindlichkeit während und nach der Abbindereaktion. Während die Wasserempfindlichkeit während der Abbindereaktion aus prinzipiellen Gründen kaum zu vermeiden ist, kann die Wasserfestigkeit der Massen nach beendeter Verfestigung in zweierlei Weise verbessert werden, nämlich durch Verwendung einer sehr reaktiven Pulverzusammensetzung oder durch Verwendung einer besonders reaktiven Abbindeflüssigkeit. Besonders gute Ergebnisse erhält man naturgemäß durch Kombination beider Möglichkeiten. Dabei findet jedoch in jedem Fall die Reaktion so rasch statt, daß die Verarbeitungszeit, d. h. die Zeit, die zum Einbringen und Modellieren des Zements in der Kavität zur Verfügung steht, äußerst kurz ist. In vielen Fällen tritt die Erhärtung bereits während des Anmischens ein. Um eine ausreichende Verarbeitungszeit zu erhalten, wird daher mit weniger reaktiven Pulvern und weniger reaktiven Flüssigkeiten gearbeitet, so daß die derzeit auf dem Markt befindlichen Präparate alle über längere Zeit wasserempfindlich sind.

Aus der DE-C 1 267 589 ist es bekannt, für die Herstellung von porösen Sinterkörpern (Filtern) dienenden Glasgrieß mit Laugen oder Säuren zu behandeln, um die Oberfläche der Glaskörper zu zerklüften.

Ziel der Erfindung war es, die Wasserempfindlichkeit von auf dem Gebiet der Medizin oder Zahnmedizin verwertbaren Zementen auf der Basis von Glaspulvern und Polycarbonsäuren herabzusetzen und gleichzeitig eine ausreichende Verarbeitungszeit zu gewährleisten.

Es wurde nun gefunden, daß man dieses Ziel erreicht, wenn man als Pulverkomponente der Glasionomerzemente ein Calciumaluminiumfluorosilikatglas verwendet, dessen Pulverteilchen an der Oberfläche im Vergleich zur durchschnittlichen Zusammensetzung an Calciumionen verarmt sind.

Gegenstand der Erfindung ist daher das in den Patentansprüchen beschriebene Calciumaluminiumfluorosilikatglas-Pulver und seine Verwendung.

Die erfindungsgemäßen Calciumaluminiumfluorosilikatglas-Pulver bestehen neben Sauerstoff im Kernbereich der Pulverteilchen vorzugsweise aus:

| Bestandteil | Berechnet als | Gew.-% |
|---|---|---|
| Si | $SiO_2$ | 20—60 |
| Al | $Al_2O_3$ | 10—50 |
| Ca | $CaO$ | 1—40 |
| F | F | 1—40 |
| Na | $Na_2O$ | 0—10 |
| P | $P_2O_5$ | 0—10 |

und insgesamt 0—20 Gew.-%, berechnet als Oxide, an B, Bi, Zn, Mg, Sn, Ti, Zr, La oder anderen 3wertigen Lanthanoiden, K, W, Ge, sowie weiteren Zusätzen, welche die Eigenschaften nicht beeinträchtigen und physiologisch unbedenklich sind.

Vorteilhaft besteht der Kernbereich der Pulverteilchen aus:

| | |
|---|---|
| Si als $SiO_2$ | 25—50 Gew.-% |
| Al als $Al_2O_3$ | 10—40 Gew.-% |
| Ca als $CaO$ | 10—35 Gew.-% |
| F | 5—30 Gew.-% |

Na als $Na_2O$     0 – 8 Gew.-%
P als $P_2O_5$     1 – 10 Gew.-%

und 0 – 10 Gew.-% an $B_2O_3$, $Bi_2O_3$, ZnO, MgO, $SnO_2$, $TiO_2$, $ZrO_2$, $La_2O_3$ oder anderen Oxiden 3wertiger Lanthanoiden, $K_2O$, $WO_3$, $GeO_2$, sowie weiteren Zusätzen, welche die Eigenschaften nicht beeinträchtigen und physiologisch unbedenklich sind.

Besonders bevorzugt:

Si als $SiO_2$     25 – 45 Gew.-%
Al als $Al_2O_3$     20 – 40 Gew.-%
Ca als CaO     10 – 30 Gew.-%
F     10 – 30 Gew.-%
Na als $Na_2O$     1 – 8 Gew.-%
P als $P_2O_5$     1 – 10 Gew.-%

Hinsichtlich der chemischen Bruttozusammensetzung im Kernbereich der Pulverteilchen kommen somit beispielsweise die Gläser in Betracht, die in der DE-A 2 061 513 oder in der DE-B 20 65 824 beschrieben werden.

Beispiele für bevorzugte Zusammensetzungen im Kernbereich sind in der folgenden Tabelle I aufgeführt:

Tabelle I

Beispiele für die Zusammensetzung des Kernbereichs erfindungsgemäßer Glaspulver:

| Gew.-% | | | |
|---|---|---|---|
| | A | B | C | D |
| Si als $SiO_2$ | 35,0 | 27,6 | 29,0 | 45,4 |
| Al als $Al_2O_3$ | 30,4 | 26,0 | 25,1 | 35,0 |
| Ca als CaO | 14,9 | 28,8 | 24,6 | 10,1 |
| F | 17,7 | 17,0 | 23,0 | 10,4 |
| Na als $Na_2O$ | 2,7 | 2,1 | 2,2 | 6,9 |
| P als $P_2O_5$ | 6,9 | 8,3 | 5,8 | 2,4 |

Die erfindungsgemäßen Glaspulverteilchen sind an der Oberfläche an Calcium so verarmt, daß der Quotient aus dem Atomverhältnis Si/Ca an der Oberfläche der Pulverteilchen und dem Atomverhältnis Si/Ca im Kernbereich mindestens 2,0, vorzugsweise mindestens 3,0 und besonders bevorzugt mindestens 4,0 beträgt.

Die Tiefe des Verarmungsbereichs hängt von den jeweiligen praktischen Gegebenheiten, insbesondere von der angestrebten Verarbeitungszeit der aus den erfindungsgemäßen Glaspulvern hergestellten Zemente ab. In der Praxis ist es günstig, wenn sich die Calciumverarmung mindestens so weit erstreckt, daß die Verarbeitungszeit des Gemisches aus dem erfindungsgemäßen Pulver und der Polycarbonsäurelösung mindestens 1,5 Min. bei 23°C beträgt. Im allgemeinen erstreckt sich der Verarmungsbereich vorzugsweise mindestens bis zu einer Tiefe von etwa 10 nm, insbesondere von mindestens etwa 20 nm und ganz besonders von mindestens etwa 100 nm. Diese Bereiche sind bei Verwendung der erfindungsgemäßen Glaspulver für Dentalzwecke bevorzugt geeignet. Für andere Zwecke, z. B. für die Verwendung für Knochenzemente, kann der Verarmungsbereich auch größer sein und z. B. auch 200 bis 300 nm betragen. Der Calciumgehalt nimmt von der Oberfläche zum Kernbereich asymptotisch zu.

Wie später ausgeführt, werden die erfindungsgemäßen Glaspulver durch eine Oberflächenbehandlung von Glaspulvern der dem Kernbereich der erfindungsgemäßen Pulver entsprechenden Zusammensetzung hergestellt. Bei der Oberflächenbehandlung bleibt die Anzahl der Siliciumatome pro Volumen-Einheit praktisch konstant. Die wahre Veränderung in der absoluten Atomzahl pro Volumeneinheit der übrigen Atomsorten erhält man daher durch Quotientenbildung des relativen Atomanteils mit dem prozentualen Siliciumanteil, wie später an einem praktischen Beispiel dargelegt wird. Der Quotient aus dem Atomverhältnis Si/Ca an der Oberfläche der Pulverteilchen und dem Atomverhältnis Si/Ca im Kernbereich stellt daher einen brauchbaren Wert zur Charakterisierung der erfindungsgemäßen Glaspulver dar. Wie die später aufgeführten Beispiele zeigen, nähert sich das Atomverhältnis Si/Ca für die einzelnen Schichten des Glaspulvers asymptotisch dem Wert des unbehandelten Ausgangsmaterials und damit des Kernbereichs des behandelten Pulvers.

Die Oberflächenmessung zur Ermittlung der Ca-Verarmung der erfindungsgemäßen Glaspulver wird zweckmäßig durch Photoelektronen-Spektroskopie (ESCA) durchgeführt. Diese Methode wird vom R. S. Swingle II und W. M. Riggs in Critical Reviews in Analytical Chemistry, Volume 5, Issue 3, Seiten 267 – 321, 1975 und von K. Levsen in »Chemie in unserer Zeit«, 10. Jahrgang (1976), Nr. 2, S. 48 – 53 beschrieben.

Die der vorliegenden Beschreibung zugrunde liegenden Meßwerte wurden unter folgenden Meßbedingungen der ESCA-Messung ermittelt:

Gerät:
Scanning-Auger-ESCA-Spektrometer, Mod. PHI 550 der Fa. Physical Electronics Industries, München (vgl. Perkin-Elmer Prospekt ESCA/SAM, PHI Data Sheet 1052 2-79 3 M)
Anregung:
400 W Mg-Strahlung
Gitterdurchtrittsenergie:
100 eV
Zeitkonstante:
0,1 Sek.

Die erfindungsgemäßen Glaspulver weisen eine durchschnittliche Korngröße (Gewichtsmittel) von wenigstens 0,5 µm, bevorzugt wenigstens 1 µm und besonders bevorzugt mindestens 3 µm auf. Für Dentalzwecke beträgt die durchschnittliche Korngröße (Gewichtsmittel) 1 – 20 µm, bevorzugt 3 – 15 µm und besonders bevorzugt 3 – 10 µm. Die Teilchen haben eine maximale Korngröße von 150 µm, vorzugsweise 100 µm, besonders 60 µm. Für die Verwendung als dentaler Befestigungszement beträgt die maximale Teilchengröße 25 µm, vorzugsweise 20 µm. Zur Erzielung guter mechanischer Eigenschaften ist in üblicher Weise eine nicht zu enge Korngrößenverteilung günstig, wie sie beispielsweise durch übliche Vermahlung und Absiebung der Grobanteile erreicht wird.

Die Herstellung der erfindungsgemäßen Glaspulver erfolgt ausgehend von Glaspulvern der durchschnittlichen Zusammensetzung des Kernbereichs der erfindungsgemäßen Pulver. Hierfür geeignet sind beispielsweise die in der DE-A 2 061 513 und der Tabelle I beschriebenen Glaspulver. Die als Ausgangsmaterialien eingesetzten Glaspulver werden wie üblich durch Zusammenschmelzen der Ausgangs-Komponenten bei Temperaturen oberhalb 950° C, Abschrecken und Mahlen gewonnen. Als Ausgangs-Komponenten können beispielsweise die in der DE-A 2 061 513 angegebenen Verbindungen in entsprechenden Mengenbereichen eingesetzt werden.

Die so erhaltenen Pulver werden dann einer Oberflächenbehandlung unterzogen. Die erfindungsgemäßen Pulver sind z. B. erhältlich durch Entfernung von Ca mittels geeigneter chemischer Agentien.

Nach einer Ausführungsform der Erfindung werden die Ausgangs-Glaspulver mit Säure oberflächlich behandelt, vorzugsweise bei Raumtemperatur. Dabei werden saure Gruppen enthaltende Substanzen eingesetzt, bevorzugt solche, die lösliche Calciumsalze bilden. Eine geringe Wasserlöslichkeit der entsprechenden Calciumsalze kann dabei in gewissen Grenzen durch eine große Flüssigkeitsmenge pro Pulvereinheit kompensiert werden. Die Reaktionszeit beträgt je nach Säurestärke und Konzentration der eingesetzten Säure zwischen wenigen Minuten und einigen Tagen.

Beispielsweise können zur Herstellung der erfindungsgemäßen Pulver Salzsäure, Schwefelsäure, Salpetersäure, Essigsäure, Propionsäure und Perchlorsäure verwendet werden.

Die Säuren werden in einer Konzentration von 0,01 bis 10 Gew.-%, vorzugsweise von 0,05 bis 3 Gew.-% eingesetzt.

Nach der entsprechenden Reaktionszeit werden die Pulver von der Lösung separiert und gründlich ausgewaschen, so daß sich praktisch keine löslichen Calciumsalze mehr auf der Oberfläche der Pulverteilchen befinden. Abschließend wird das Pulver getrocknet, vorzugsweise oberhalb 70° C, und auf die gewünschten Korngrößenbereiche gesiebt.

Je stärker die verwendete Säure ist und je länger eine gegebene Säure auf das Pulver einwirkt, desto länger gestaltet sich die Verarbeitungszeit nach dem Anmischen mit der Anrührflüssigkeit.

Die günstige Oberflächenbeschaffenheit der erfindungsgemäßen Pulver erlaubt die Anwendung eines besonders hohen Pulver/Flüssigkeits-Verhältnisses im angemischten Zement, wodurch hohe Festigkeitswerte des ausgehärteten Materials erreicht werden. In gleicher Weise wirkt die Möglichkeit der Verwendung einer besonders reaktiven Anmischflüssigkeit. Weiterhin kann die Verarbeitungszeit eines erfindungsgemäßen Zementes den Bedürfnissen des Anwenders angepaßt werden. Die Länge der Verarbeitungszeit beeinflußt dabei die anschließende Aushärtungszeit kaum, so daß auch bei langen Verarbeitungszeiten eine schnelle Verfestigung und frühzeitige Wasserunempfindlichkeit eintritt.

Die erfindungsgemäßen Glaspulver eignen sich somit besonders zur Verwendung in Zahn- und Knochenzementen. Die Erfindung betrifft daher auch die Verwendung der erfindungsgemäßen Glaspulver zur Herstellung von selbsthärtenden Glasionomerzementen.

Die erfindungsgemäßen Pulver können zu Zahnsegmenten oder Knochenzementen mit den herkömmlichen wäßrigen Polycarbonsäurelösungen angemischt werden, wie sie beispielsweise in der DE-A 2 061 513, der DE-A 2 439 882, sowie der DE-A 2 101 889 beschrieben werden. Geeignete Polycarbonsäuren sind Polymaleinsäure, Polyacrylsäure, sowie Gemische davon, oder Copolymere, insbesondere Maleinsäure-Acrylsäure-Copolymere und/oder Acrylsäure-Itaconsäure-Copolymere. Es versteht sich von selbst, daß man bei Verwendung eines extrem reaktiven Glaspulvers eine weniger reaktive Polycarbonsäure verwendet, um eine zufriedenstellende Erhärtungscharakteristik zu erhalten.

Zur Beschleunigung und Verbesserung der Aushärtung dieser Glas-Ionomer-Zemente können beim Anmischen in bekannter Weise (DE-A 2 319 715) chelatbildende Mittel zugesetzt werden. Vorzugsweise wird als Chelatbildner Weinsäure in den üblichen Konzentrationen bereits der Anmischflüssigkeit zugesetzt.

Statt der üblichen Verwendung des erfindungsgemäßen Glaspulvers, nämlich mit der wäßrigen Polycarbonsäurelösung als Anmischflüssigkeit, kann das Glaspulver auch mit der trockenen pulverisierten Polycarbonsäure im entsprechenden Verhältnis vorgemischt werden, da die festen Substanzen nicht miteinander reagieren. Als Anmischflüssigkeit dient dann Wasser, vorzugsweise eine wäßrige Lösung eines Chelatbildners, insbesondere Weinsäure, gegebenenfalls mit üblichen Zusätzen, wie bakteriostatischen Mitteln.

Zur Vermeidung von Dosierfehlern und zur Erzielung optimaler mechanischer Eigenschaften ist die Verwendung der erfindungsgemäßen Pulver in prädosierter Form vorteilhaft. In einer Ausführungsform wird das Glaspulver in Kunststoffbehälter portioniert. Der Zement kann dann entweder in diesen Kunststoffkapseln mechanisch oder nach Entleeren der Behälter von Hand angemischt werden. Die Dosierung der wäßrigen Polycarbonsäurelösung geschieht in diesem Falle z. B. mit einer Tropfflasche oder einer Spritze. Besonders geeignet ist die Verwendung des erfindungsgemäßen Pulvers in sogenannten Schüttelkapseln, z. B. entsprechend der DE-A 2 324 296. Das Pulver wird dabei in einer Hauptkammer prädosiert bereitgehalten, während die Flüssigkeit in einem separaten Kissen unter einer seitlichen Spange enthalten ist. Durch Ausüben von Druck auf diese Spange wird die Flüssigkeit durch eine Bohrung in die Hauptkammer gespritzt und steht dann für den mechanischen Anmischvorgang zur Verfügung. In beiden Kapselarten kann statt des reinen Glaspulvers auch das Gemisch aus Glaspulver und trockener Polycarbonsäure vordosiert eingesetzt werden. Als flüssige Komponente kommt das Wasser oder eine wäßrige Lösung eines Chelatbildners, insbesondere Weinsäure, zum Einsatz.

Die Verwendung des Gemisches aus erfindungsgemäßem Glaspulver und trockener Polycarbonsäure ist besonders vorteilhaft, wenn diese Mischung in Tablettenform konfektioniert wird. Dazu wird die trockene Polycarbonsäure in fein zerteilter Form und nach Entfernung von Grobanteilen verwendet. Nach gründlichem Vermischen dieses Polycarbonsäurepulvers mit dem erfindungsgemäßen Glaspulver können in einer üblichen Tablettiermaschine Tabletten gepreßt werden. Der Preßdruck muß dabei so gewählt werden, daß die Tabletten nach Zugabe der Anmischflüssigkeit (Wasser oder z. B. wäßrige Weinsäurelösung) noch gut zu einem Zement verarbeitet werden können und andererseits eine ausreichende mechanische Stabilität für Transportzwecke gewährleistet ist. Derart hergestellte Tabletten ermöglichen nach kurzem Anlösen z. B. in der entsprechenden Menge Weinsäurelösung ein besonders einfaches Vermischen zur Zementpaste. Die Anmischflüssigkeit kann z. B. aus einer Tropfflasche oder Spritze zudosiert werden.

Die folgenden Beispiele dienen zur Erläuterung der Erfindung:

## Beispiel 1

Nach an sich bekannten Verfahren (z. B. DE-A 2 061 513) wird ein Glaspulver hergestellt, bestehend aus:

| | |
|---|---|
| Si als $SiO_2$ | 35,0 Gew.-% |
| Al als $Al_2O_3$ | 30,4 Gew.-% |
| Ca als CaO | 14,9 Gew.-% |
| Na als $Na_2O$ | 2,7 Gew.-% |
| P als $P_2O_5$ | 6,9 Gew.-% |
| F | 17,7 Gew.-% |

Durch Vermahlen der abgeschreckten Glasmasse in einer Kugelmühle erhält man ein feinkörniges Glaspulver.

100 g dieses Pulvers werden in 1000 g 0,15%iger wäßriger HCl-Lösung aufgeschlämmt und 1 Stunde intensiv gerührt. Anschließend wird abfiltriert, chloridfrei gewaschen und 2 Stunden bei 120°C getrocknet und auf eine Korngröße von <60 μm gesiebt. Das Pulver hat dann ein Gewichtsmittel der Korngröße von ca. 8 μm.

Aus der folgenden Tabelle II sind die Ergebnisse von ESCA-Messungen der Atomzusammensetzung der erfindungsgemäß behandelten Probe in verschiedenen Schichttiefen ersichtlich:

Tabelle II

Atom-% (genormt auf $\Sigma = 100\%$)

|  | Si/Ca | O | Si | Al | Ca | F | P |
|---|---|---|---|---|---|---|---|
| Oberfläche | 18,0 | 53,7 | 27,0 | 11,8 | 1,5 | 2,4 | 3,5 |
| ~ 2,5 nm | 16,9 | 51,5 | 27,0 | 13,7 | 1,6 | 3,2 | 3,1 |
| ~ 5 nm | 15,4 | 50,0 | 27,8 | 14,1 | 1,8 | 3,5 | 2,7 |
| ~ 10 nm | 9,8 | 49,0 | 25,5 | 15,8 | 2,6 | 5,0 | 2,1 |
| ~ 15 nm | 7,6 | 47,6 | 24,4 | 16,5 | 3,2 | 6,2 | 2,0 |
| ~ 20 nm | 6,2 | 47,0 | 23,7 | 16,8 | 3,8 | 7,0 | 1,7 |
| ~ 30 nm | 5,2 | 47,2 | 22,5 | 16,9 | 4,3 | 7,5 | 1,5 |
| ~ 50 nm | 4,2 | 45,8 | 20,9 | 18,7 | 5,0 | 8,4 | 1,2 |
| ~ 75 nm | 3,5 | 44,5 | 19,8 | 19,9 | 5,7 | 10,0 | 1,0 |
| ~100 nm | 3,2 | 44,0 | 19,6 | 18,9 | 6,2 | 10,1 | 1,1 |
| ~125 nm | 3,1 | 42,4 | 19,9 | 20,2 | 6,4 | 10,0 | 1,1 |
| ~150 nm | 2,9 | 41,9 | 19,3 | 20,6 | 6,6 | 10,5 | 1,0 |
| ~200 nm | 2,8 | 41,8 | 19,0 | 21,0 | 6,9 | 10,7 | 0,7 |
| ~250 nm | 2,5 | 41,2 | 18,4 | 20,9 | 7,3 | 11,3 | 0,9 |

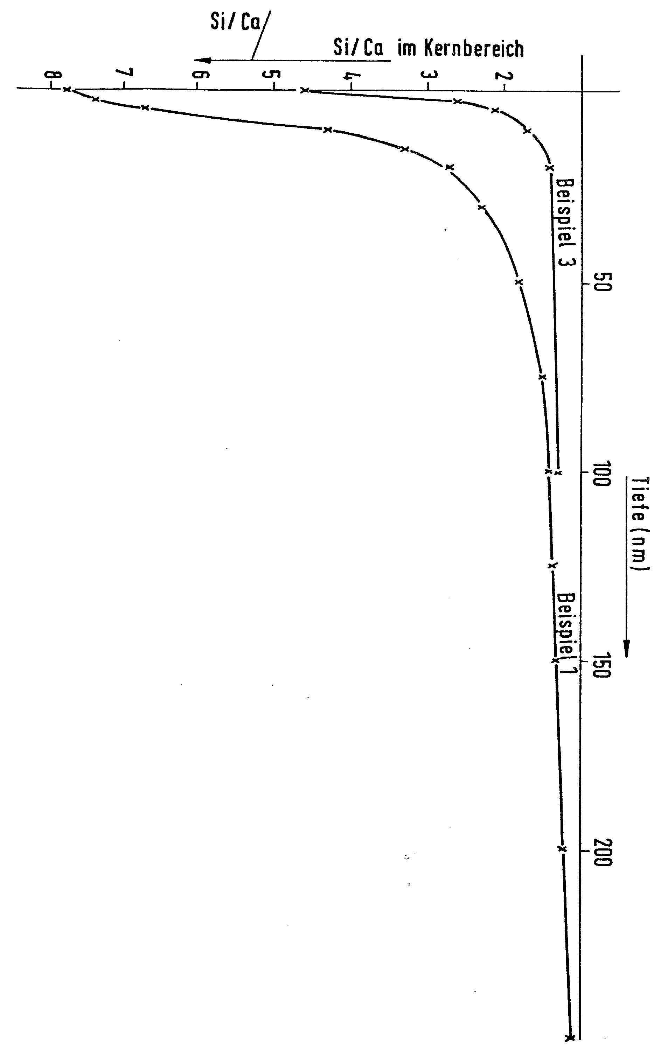

Aus der Tabelle und der beigefügten Figur ist ersichtlich, daß sich das Si/Ca-Verhältnis von der Oberfläche zur Teilchenmitte asymptotisch einem Grenzwert im Kernbereich von etwa 2,3 nähert. Der Quotient aus dem Si/Ca-Atomverhältnis an der Oberfläche und dem des Kernbereichs liegt daher in diesem Beispiel bei etwa 7,8.

## Beispiel 2

Das im Beispiel 1 eingesetzte Pulver wird nach der in Beispiel 1 beschriebenen Weise behandelt, dabei werden jedoch statt Salzsäure die in der Tabelle aufgeführten Säuren mit den entsprechenden Rührzeiten angewendet.

| Säure | Säure-konzentration | Rührzeit |
|---|---|---|
| Schwefelsäure | 0,5% | 1 Stunde |
| Essigsäure | 0,3% | 20 Stunden |
| Salpetersäure | 0,5% | 1 Stunde |
| Essigsäure | 3,0% | 1 Stunde |
| Propionsäure | 2,0% | 1 Stunde |
| Perchlorsäure | 0,3% | 1 Stunde |

Man erhält in jedem Falle Glaspulver, die etwa dem des Beispiels 1 entsprechen.

### Beispiel 3

10 g eines käuflichen Glasionomerzementpulvers der Zusammensetzung nach Tab. I C (De Trey, Aspa, A. D. International, London, England, Gewichtsmittel der Korngröße ca. 6,5 μm) werden entsprechend Beispiel 1 mit 100 g 0,4%iger wäßriger Salzsäurelösung behandelt.

Die atomare Zusammensetzung dieses Pulvers wurde in verschiedenen Schichttiefen nach der ESCA-Methode bestimmt. Die Ergebnisse sind in der folgenden Tabelle III aufgeführt:

Tabelle III

Atom-% (genormt auf $\Sigma = 100\%$)

| | Si/Ca | O | Si | Al | Ca | F | P |
|---|---|---|---|---|---|---|---|
| Oberfläche | 3,2 | 51,0 | 16,9 | 12,0 | 5,3 | 7,8 | 7,0 |
| 2,5 nm | 1,8 | 43,1 | 16,2 | 12,6 | 8,8 | 13,4 | 5,9 |
| 5 nm | 1,5 | 40,5 | 15,9 | 12,5 | 10,5 | 15,7 | 4,9 |
| 10 nm | 1,2 | 37,4 | 15,1 | 11,6 | 12,6 | 19,1 | 4,2 |
| 20 nm | 1,0 | 34,3 | 14,4 | 11,8 | 14,6 | 21,6 | 3,2 |
| 100 nm | 0,9 | 32,1 | 15,0 | 12,1 | 16,4 | 23,1 | 1,4 |

Aus der Tabelle und der beigefügten Figur ist ersichtlich, daß sich das Si/Ca-Verhältnis von der Oberfläche zur Teilchenmitte asymptotisch einem Grenzwert im Kernbereich von 0,7 nähert. Der Quotient aus dem Atomverhältnis Si/Ca an der Oberfläche und dem des Kernbereichs liegt daher bei 4,6.

### Beispiel 4

Das im Beispiel 1 eingesetzte unbehandelte Pulver gemäß dem Stand der Technik und das erfindungsgemäße Pulver nach Beispiel 1 werden jeweils durch ein Sieb mit Maschenweite von 60 μm gesiebt. Die Pulver werden mit käuflicher Anmischflüssigkeit für Glasionomerzemente (Acrylsäure/Itaconsäure-Copolymeres, De Trey, Aspa Flüssigkeit, A. D. International, London, England) im Gewichtsverhältnis 3,5 : 1 angeteigt. Die Aushärtung wird anschließend auf einem Rheometer bei 28°C verfolgt.

| | |
|---|---|
| unbehandeltes Glaspulver nach Beispiel 1 (Stand der Technik) | Verarbeitungszeit: sofortiger Beginn der Aushärtung |

7

behandeltes Pulver nach  Verarbeitungszeit:
Beispiel 1                3 Min. 30 Sek.
(erfindungsgemäß)         Aushärtungszeit:
                          6 Min. 10 Sek.

## Beispiel 5

Das erfindungsgemäße Pulver nach Beispiel 1 wird im Gewichtsverhältnis 3,5 : 1 mit einer ca. 45%igen wäßrigen Polymaleinsäurelösung (nach DE-A 2 101 889) angeteigt. Der Polymaleinsäurelösung waren zuvor noch 10 Gew.-% Weinsäure zugesetzt worden. Die Aushärtung der Zementmischung wurde an einem Rheometer verfolgt:

Verarbeitungszeit:       2 Minuten
Aushärtungszeit:         4 Minuten

Man erhält eine günstige Verarbeitungszeit bei anschließend rascher Aushärtung.

## Beispiel 6

Die Wasserempfindlichkeit der Glasionomerzemente wird nach folgender Methode bestimmt:

Zylindrische Formkörper von 10 mm Ø und 2,5 mm Höhe werden mit dem frisch angemischten, zu prüfenden Material gefüllt und 10 Min. nach dem Anmischen in eine Lösung von 1% Patentblau in Wasser gegeben (Raumtemperatur). In der Lösung werden die Formkörper 10 Minuten belassen, danach abgespült und abgetrocknet. Der Formkörper wird an der Rundseite mit feinem Schleifpapier zu ca. 1/3 plan abgeschliffen.

Die Dicke der in den Körper eingedrungenen Farbschicht wird unter dem Mikroskop vermessen. Durch Zufallsauswahl werden 5 Meßwerte bestimmt und daraus der Mittelwert gebildet.

| Pulver | Flüssigkeit | Pulver/Flüssig-keits-Verhältnis | Farb-eindringtiefe ($\mu$m) |
|---|---|---|---|
| Nach Beispiel 1 (erfindungsgemäß) | wäßrige Lösung von Acrylsäure/Itacon-säure-Copolymeren (handelsüblich: De Trey »Aspa«) | 3,5 | 0 |
| Tab I, C (handelsüblich: De Trey, »Aspa«) | desgl. | 3,0 (nach Her-stellervorschrift) | 125 |
| Nach Beispiel 1 (erfindungsgemäß) | handelsüblich (G. C. »Fuji«) | 3,5 | 0 |
| Tab I, D (handelsüblich: G. C. »Fuji«) | handelsüblich (G. C. »Fuji«) | 2,3 (nach Her-stellervorschrift) | 65 |

## Beispiel 7

Das nach Beispiel 1 hergestellte Pulver ergibt nach Zusatz von geeigneten Farbpigmenten in den üblichen Mengen ein Pulver, das als Zementmischung geeignet ist, defekte Zahnteile zu ersetzen. Als Anmischflüssigkeit wird eine ca. 45%ige wäßrige Polymaleinsäurelösung (nach DE-A 2 101 889), der 10 Gew.-% Weinsäure zugesetzt wurden, verwendet.

Nach dem Anmischen dieses Pulver mit der Flüssigkeit im Gewichtsverhältnis 3,5 : 1 wird eine Paste guter Konsistenz erhalten, die nach dem Aushärten einen Zement zahnähnlicher Transparenz ergibt und folgende physikalische Werte aufweist:

Druckfestigkeit:         175 MPa
Oberflächenhärte:        400 MPa
Verarbeitungszeit:       2 Minuten

8

| Aushärtungszeit: | 4 Minuten |
|---|---|
| Wassereindringtiefe: (gemäß Beispiel 6) | 0 µm |

### Beispiel 8

Nach an sich bekannten Verfahren (z. B. DE-A 2 061 513) wird ein Glaspulver hergestellt, bestehend aus:

| Si als $SiO_2$ | 27,6 Gew.-% |
|---|---|
| Al als $Al_2O_3$ | 26,0 Gew.-% |
| Ca als CaO | 28,8 Gew.-% |
| Na als $Na_2O$ | 2,1 Gew.-% |
| P als $P_2O_5$ | 8,3 Gew.-% |
| F | 17,0 Gew.-% |

Durch Vermahlen der abgekühlten Glasmasse in einer Kugelmühle erhält man ein feinkörniges Glaspulver.

Das erhaltene Pulver wird nach Beispiel 1 mit 0,1%iger wäßriger Salzsäurelösung 1 Stunde behandelt. Anschließend wird es durch ein Sieb der Maschenweite 20 µm gesiebt. Das erhaltene Pulver ist zu einer Verwendung als Befestigungszement für Zahnersatzteile geeignet.

Dazu wird das erhaltene Pulver im Gewichtsverhältnis 1,8 : 1 mit einer handelsüblichen Anmischflüssigkeit für Glasionomer-Befestigungszemente (Chem. Bond, A. D. International, London, England) angemischt. Das dünnviskose Gemisch bleibt bei Raumtemperatur ca. 3 Minuten verarbeitbar und ist nach 8 Minuten ausgehärtet.

### Beispiel 9

100 g des in Beispiel 7 beschriebenen erfindungsgemäßen Pulvers werden mit 10,5 g eines trockenen Polymaleinsäurepulvers (<60 µm), (hergestellt z. B. nach DE-A 2 101 889) vermischt. Aus der homogenen Mischung werden in üblicher Weise Tabletten von ca. 200 mg hergestellt (Ø 8 mm, Dicke ca. 2 mm).

Eine dieser Tabletten wird kurz in 34 mg 14%iger Weinsäurelösung eingeweicht. Nach Anspateln unter leichtem Druck erhält man eine homogene Paste guter Konsistenz, die als selbsthärtender Zahnzement geeignet ist.

### Beispiel 10

In die Mischkammer von Schüttelkapseln gemäß DE-A 1 910 885 wird das in Beispiel 7 beschriebene erfindungsgemäße Pulver in Portionen von 280 mg eingewogen. Als getrennte Kammer enthält diese Kapsel ein Folienkissen aus kunststoffkaschiertem Aluminium, das mit 96 mg einer ca. 45%igen wäßrigen Polymaleinsäurelösung gefüllt ist. Wenn die so vorbereitete Kapsel, wie in der o. g. Offenlegungsschrift beschrieben, benützt wird, erhält man nach dem Anmischen mit einem mechanischen Mischer einen Zement, der als Dauerfüllungsmaterial für Zahnkavitäten geeignet ist.

### Beispiel 11

Ein Gemisch aus 285 mg erfindungsgemäßen Pulver entsprechend Beispiel 7 und 35 mg eines getrockneten Maleinsäurepolymeren werden in die Mischkammer einer Schüttelkapsel, gemäß DE-A 1 910 885, gefüllt. Als getrennte Kammer enthält diese Kapsel ein Folienkissen aus kunststoffkaschiertem Aluminium, das mit 54 mg einer 14%igen Weinsäurelösung gefüllt ist. Verfährt man weiter wie in Beispiel 10 beschrieben, so erhält man wiederum einen Zement, der als dentales Dauerfüllungsmaterial geeignet ist.

### Beispiel 12

100 g des in Beispiel 1 eingesetzten Pulvers werden mit 1000 g 3%iger wäßriger Essigsäurelösung 2 Stunden intensiv gerührt. Anschließend wird abfiltriert, gründlich nachgewaschen und 2 Stunden bei 120°C getrocknet und auf eine Korngröße von <60 µm gesiebt.

Nach Anmischen im Gewichtsverhältnis 2,5 : 1 mit ca. 45%iger Polymaleinsäurelösung erhält man eine Zementmasse, die ca. 5 Minuten verarbeitbar bleibt und nach 8 Minuten ausgehärtet ist.

Diese Zementmasse eignet sich besonders als Knochenzement, z. B. zur Befestigung von Hüftgelenksprothesen.

**Patentansprüche**

1. Calciumaluminiumfluorosilikatglas-Pulver mit einer durchschnittlichen Korngröße von wenigstens 0,5 μm, dadurch gekennzeichnet, daß die Pulverteilchen an der Oberfläche an Calcium so verarmt sind, daß der Quotient aus dem Atomverhältnis Si/Ca an der Oberfläche der Pulverteilchen und dem Atomverhältnis Si/Ca im Kernbereich mindestens 2,0 ist.

2. Verwendung der Calciumaluminiumfluorosilikatglas-Pulver nach Anspruch 1 zur Herstellung von selbsthärtenden Glasionomerzementen.

**Claims**

1. Calcium aluminium fluorosilicate glass powder having an average particle size of at least 0.5 microns, characterized in that the powder particles are so depleted of calcium at their surface that the quotient of the atomic ration Si/Ca at the surface of the powder particles and the atomic ratio Si/Ca in the core region is at least 2.0.

2. Use of the calcium aluminium fluorosilicate glass powder according to claim 1 for preparing self-hardening glass ionomer cements.

**Revendications**

1. Poudre de verre au fluosilicate de calcium-aluminium, formée de grains ayant une grosseur moyenne d'au moins 0,5 μm, caractérisée en ce que les particules de la poudre présentent en surface un appauvrissement en calcium tel que le quotient du rapport atomique Si/Ca, à la surface des particules par le rapport atomique Si/Ca, dans la région du coeur, soit d'au moins 2,0.

2. Application de la poudre de verre au fluosilicate de calcium-aluminium suivant la revendication 1, à la préparation de ciments ionomères de verre, durcissant spontanément.